# EUROPÄISCHE PATENTSCHRIFT

(11) **EP 2 461 779 B1**
(45) Veröffentlichungstag und Bekanntmachung des Hinweises auf die Patenterteilung: **19.03.2014**
(21) Anmeldenummer: 10740167.1
(22) Anmeldetag: 30.07.2010
(51) Int. Cl.: A61F 13/15, A61F 13/56, A61F 13/62

(54) **GEFALTETER INKONTINENZARTIKEL**
FOLDED INCONTINENCE ARTICLE
ARTICLE PLIÉ POUR INCONTINENCE

(30) Priorität: 08.08.2009 DE 102009036796
(43) Veröffentlichungstag der Anmeldung: 13.06.2012
(73) Patentinhaber: Paul Hartmann AG, 89522 Heidenheim (DE)
(72) Erfinder: König, Michael, 89367 Waldstetten (DE); GUNESCH, Hansgeorg, 89537 Glengen (DE); WENZEL, Benjamin, 85748 Garching (DE); KESSELMEIER, Rüdiger, 89542 Herbrechtingen (DE); KOCH, Christian, 89429 Bachhagel (DE)
(86) Internationale Anmeldenummer: PCT/EP2010/004677
(87) Internationale Veröffentlichungsnummer: WO 2011/018167

(56) Entgegenhaltungen:
- EP-A2- 1 269 949
- EP-B1- 1 166 735
- WO-A1-2005/102241
- WO-A1-2007/014623
- WO-A1-2008/141834
- US-B1- 6 743 213

## Beschreibung

Die vorliegende Erfindung betrifft gefaltete, absorbierende Inkontinenzartikel des offenen Typs für inkontinente Erwachsene, und ein Verfahren zur Faltung absorbierender Inkontinenzartikel.

DE-102005035544-A1 beschreibt bereits einen Inkontinenzartikel mit an Seitenrändern des Hauptteils angestückten Materialabschnitten, die häufig auch als Windelohren bezeichnet werden, die im Randbereich Verschlusselemente tragen, wobei die Windelohren um wenigstens zwei Falzlinien auf sich selbst gefaltet sind und auf die körperzugewandte Seite des Hauptteils eingeschlagen sind, um eine übereinander eingeschlagene Anordnung zu bilden, die an einer ersten Fügestelle lösbar fixiert ist.

EP-1005316-B1 beschreibt die z-förmige Faltung der Windelohren einer Windel des offenen Typs, bei der zunächst das äußere Ende der Ohren nach hinten um mindestens die Verschlussflächenbreite gefaltet wird und anschließend in dieser Konfiguration nach vorne um die doppelte Verschlussflächenbreite zurückgefaltet wird, so dass die Verschlusselemente in dieser gefalteten Konfiguration nicht von einer Materiallage verdeckt sind und für den Anwender sofort sichtbar sind.

EP-1166735-B1 beschreibt ebenfalls eine z-förmige bzw. invers z-förmige Faltung der hinteren Windelohren, wobei die erste Faltung nach innen auf das Windelchassis erfolgt und anschließend das freie Ende des Seitenteils so ein- oder mehrfach nach außen umgefaltet wird, dass dieses im gefalteten Zustand nach außen weist und sich beim Anlegen der Windel - insbesondere an bettlägerige Personen - leicht entfalten lässt.

WO-2005/110321-A1 beschreibt die Faltung von absorbierenden Hygieneartikeln entlang erster und zweiter longitudinaler Falzlinien, derart, dass die Außenkanten der Windelohren über die Falzlinie hinaus reichen, so dass der gefaltete Artikel leicht zu entfalten ist, da die die Verschlusselemente tragende Außenkante direkt zu erreichen ist.

WO-2007/058761-A1 beschreibt die Herstellung von absorbierenden Hygieneartikeln mit separat angefügten Windelohren bei hohen Maschinengeschwindigkeiten ohne dass der Artikel oder die Verschlusselemente unbeabsichtigt zerknittert oder gefaltet werden, dadurch dass eine Faltung vorgenommen wird, die die Windelohren und Verschlusselemente schützt. Dazu wird das Windelohr, das auf seiner im angelegten Zustand nach innen liegenden Seite ein Verschlusselement trägt, an einer Längsachse auf die Innenseite des Chassis umgeschlagen und anschließend wird an einer zweiten Längsachse der gesamte Seitenteil auf das Chassis umgeschlagen, wobei die zweite Faltbreite mindestens so groß ist, wie die erste, damit der erste nach innen geschlagene Bereich durch die zweite Faltung nicht erneut gefaltet wird.

DE-102004021353-A1 offenbart einen absorbierenden Inkontinenzartikel mit einem Hauptteil, bestehend aus einem Vorderbereich, einem Rückbereich und einem in Längsrichtung dazwischen liegenden zwischen den Beinen eines Benutzers zu liegen kommenden Schrittbereich, wobei der Hauptteil einen Saugkörper umfasst, und mit an den Rückbereich angefügten hinteren Materialabschnitten und mit an den Vorderbereich angefügten vorderen Materialabschnitten, wobei sich die hinteren und vorderen Materialabschnitten in Querrichtung über seitliche Längsränder des Hauptteils hinaus erstrecken, wobei die Materialabschnitte eine im Anwendungszustand auf der körperzugewandten Seite liegende Innenseite und eine im Anwendungszustand auf der körperabgewandten Seite liegende Außenseite haben und wobei hintere Materialabschnitte an einem äußeren Randbereich der hinteren Materialabschnitte Verschlusselemente aufweisen, wobei hintere und vordere Materialabschnitte den Vorderbereich und den Rückbereich im Anwendungszustand des Artikels miteinander verbinden und wobei die Materialabschnitte vor Ingebrauchnahme des zusammengefalteten Artikels auf sich selbst und nach innen auf die körperzugewandte Seite des Rückbereichs des Hauptteils gefaltet sind.

Ausgehend von diesem Stand der Technik besteht das Problem der gefalteten Inkontinenzartikel darin, dass bei hohen Fertigungsgeschwindigkeiten im Herstellungsprozess der Anteil nicht spezifikationskonform hergestellter Produkte steigt. Als nicht spezifikationskonform hergestellt werden Produkte bezeichnet, die nicht die herstellerseitig festgelegten Vorgaben bzgl. der Qualität der Produkte erfüllen. Die Produktqualität wird bei Erhöhung der Fertigungsgeschwindigkeit dadurch beeinträchtigt, dass sich die auf den gefalteten Windelohren angebrachten Verschlusselemente während der Fertigung ungewollt öffnen. Wird der Inkontinenzartikel mitsamt den gefalteten Windelohren und den ungewollt geöffneten Verschlusselementen im nächsten Fertigungsschritt längs- und/oder quer gefaltet, so kommt das geöffnete oder teilgeöffnete Verschlusselement in Kontakt mit anderen Materiallagen des Inkontinenzartikels und wird auf diesen ungewollt fixiert. Dies führt dazu, dass der Inkontinenzartikel nicht mehr in gewohnter Weise entfaltet werden kann und gegebenenfalls die Verschlusselemente für eine sinngemäße Fixierung nicht mehr brauchbar sind. Würden derartige Inkontinenzartikel nicht aus dem Produktionsprozess entfernt, so würde die ungewollte Fixierung bei der anschließenden Verpackung der Inkontinenzartikel unter Druck in einen Kunststofffolienbeutel weiter verfestigt, so dass derartige Produkte unbrauchbar werden, da sie sich nicht mehr einwandfrei öffnen und anlegen lassen.

Die Aufgabe der vorliegenden Erfindung besteht darin, absorbierende Inkontinenzartikel des offenen Typs mit angefügten Windelohren bei hoher Fertigungsgeschwindigkeit ohne Beeinträchtigung der Funktionalität der Verschlussmittel produzieren zu können. Die Inkontinenzartikel sollen außerdem in einer benutzerfreundlichen Anordnung zur Verfügung gestellt werden.

Diese Aufgabe wird bei einem Inkontinenzartikel des offenen Typs mit angefügten Windelohren (Seitenklappen oder -teile bildenden Materialabschnitten) erfindungsgemäß dadurch gelöst, dass in einer gefalteten Anordnung eines hinteren Materialabschnittes, die Innenseite des Randbereichs des Materialabschnitts in Richtung auf die körperzugewandte Seite (also die Innenseite) des Rückbereichs des Hauptteils orientiert ist. Hierbei umfasst der zu unterst liegende gefaltete Teilabschnitt des Materialabschnitts den Randbereich des Materialabschnitts, wobei der Randbereich Verschlusselemente aufweist, die eine Verschlusselementlasche umfassen, welche vor Gebrauch auf die Innenseite des Materialabschnitts zurückgefaltet ist, wobei ein äußerer Seitenrand des Teilabschnittes gegenüber den darüber gefalteten Teilabschnitten in Querrichtung um das Maß D übersteht, sich also über die eigentliche Falte hinaus erstreckt. Die Verschlusselementlaschen wirken zum Schließen des Inkontinenzartikels in Gebrauch mit einer Außenseite des Vorderbereichs des Hauptteils und/oder der Materialabschnitte im Vorderbereich lösbar haftend zusammen. Hierzu können die Verschlusselementlaschen beispielsweise eine Haftkleberzone oder mechanische Verschlusshilfen wie Kletthaken aufweisen.

Die Erfinder haben erkannt, dass der Auslöser für das ungewollte Öffnen der Verschlusselementlaschen im Fertigungsprozess eine Luftzug-Kraft ist, die auf die Verschlusselementlaschen wirkt und die mit steigender Fertigungsgeschwindigkeit ansteigt. Die Gefahr des Einwirkens dieser Luftzug-Kraft ist insbesondere zu dem Zeitpunkt gegeben, zu dem die hinteren Materialabschnitte in der gefalteten Anordnung bereits auf die Innenseite des Hauptteils eingeschlagen sind und insbesondere unmittelbar bevor oder während der Inkontinenzartikel anschließend quer zu seiner Längsrichtung gefaltet wird. Wenn diese Luftzug-Kraft die Haftkraft der Verschlusselementlasche auf der Innenseite des Materialabschnitts, auf die es zurückgeschlagen wurde, übersteigt, so öffnet sich das Verschlusselement ungewollt. Wie oben beschrieben sind derartige Inkontinenzartikel häufig völlig unbrauchbar.

Die Erfinder haben weiterhin erkannt, dass die Verschlusselemente durch die erfindungsgemäße Anordnung gegen die auftretende Luftzug-Kraft im Fertigungsprozess weitestgehend geschützt sind. Dies wird anhand der Figuren noch näher erläutert werden. Dadurch, dass ein zu unterst liegender gefalteter Teilabschnitt des hinteren Materialabschnitts den Randbereich des Materialabschnitts umfasst und der Randbereich die Verschlusselemente aufweist und ein äußerer Seitenrand des Teilabschnittes gegenüber den darüber gefalteten Teilabschnitten in Querrichtung um das Maß D übersteht, sich also über die eigentliche Falte hinaus erstreckt, ist sichergestellt, dass der Benutzer die hinteren Materialabschnitte als solche erkennt und komfortabel ergreifen und entfalten kann.

Das Maß D beträgt hierbei vorzugsweise mindestens 10 % und höchstens 90 % der Breite des zu unterst liegenden Teilabschnitts, weiterhin bevorzugt mindestens 40 % und höchstens 60 %. Der Überstand um das Maß D beträgt vorzugsweise mindestens 6 mm und höchstens 54 mm, weiterhin bevorzugt mindestens 24 mm und höchstens 36 mm.

Die hinteren Materialabschnitte sind vor Ingebrauchnahme des zusammengefalteten Artikels vorzugsweise zickzackförmig auf sich selbst gefaltet, bevorzugt an zu der Längsrichtung parallel verlaufenden Falzlinien, vorzugsweise derart, dass die Falzlinien die Materialabschnitte in drei Teilabschnitte unterteilen, wobei die Breite L3 des mittleren Teilabschnitts kleiner ist als die Breiten L2 und L4 der zum mittleren Teilabschnitt benachbarten Teilabschnitte. Vorzugsweise beträgt das Verhältnis der Breiten L2, L3, L4 der Teilabschnitte 2:1:2. Dabei schneiden die Falzlinien vorzugsweise nicht die auf die Innenseite der Materialabschnitte zurück gefalteten Verschlusselementlaschen, so dass die Breite des äußersten, einen Randbereich des jeweiligen Materialabschnittes umfassenden Teilabschnittes vorzugsweise mindestens der Breite der zurück gefalteten Verschlusselementlaschen entspricht.

Weiterhin erweist es sich als vorteilhaft, wenn an jedem der hinteren Materialabschnitte an einem jeweiligen Randbereich ein Anfassbereich vorhanden ist.

In Weiterbildung der Erfindung erweist es sich als vorteilhaft, wenn die hinteren Materialabschnitte eine größere, vorzugsweise eine um mindestens 10 %, insbesondere eine um mindestens 15% größere Flächenerstreckung (in cm²) aufweisen als die vorderen Materialabschnitte. Insbesondere kann die Länge der hinteren Materialabschnitte, also deren Erstreckung in Windellängsrichtung mindestens 13 cm, weiter insbesondere mindestens 18 cm und weiter insbesondere mindestens 22 cm betragen. Es erweist sich weiterhin als vorteilhaft, wenn die Länge der hinteren Materialabschnitte mindestens 10 %, insbesondere mindestens 15%, weiter insbesondere mindestens 20% und weiter insbesondere mindestens 22% der Gesamtlänge der Inkontinenzwegwerfwindel beträgt. Vorteilhafterweise beträgt die Gesamtlänge der Inkontinenzwegwerfwindel 50-120 cm, insbesondere 60-110 cm und weiter insbesondere 70-110 cm. Weiterhin erweist es sich als vorteilhaft, wenn die vorderen Materialabschnitte eine geringere, insbesondere eine um mindestens 5%, weiter insbesondere eine um mindestens 10%, weiter insbesondere eine um mindestens 15% und weiter insbesondere eine um höchstens 50% geringere Längserstreckung aufweisen als die hinteren Materialabschnitte., In Weiterbildung der Erfindung erweist es sich als vorteilhaft, wenn die Breite der Materialabschnitte, also die Erstreckung der Materialabschnitte in Querrichtung über den Seitenrand des Windelhauptteils hinaus 12-40 cm, insbesondere 13-30 cm, weiter insbesondere 14-25 cm beträgt. Vorzugsweise weisen die vorderen Materialabschnitte die gleiche Breite auf wie die hinteren Materialabschnitte.

Vorteilhafterweise sind auch die vorderen Materialabschnitte vor Ingebrauchnahme des zusammengefalteten Artikels auf sich selbst vorzugsweise zickzackförmig auf sich selbst gefaltet, bevorzugt an zu der Längsrichtung parallel verlaufenden Falzlinien.

Es hat sich weiter als vorteilhaft erwiesen, die vorderen und/oder hinteren Materialabschnitte aus einem Vliesstoffmaterial zu bilden. Geeignet sind insbesondere alle Vliesstoffmaterialien, die zumindest eine Rezepturkomponente auf Basis eines thermoplastischen Polymers enthalten. Die Vliesstoffe können Fasern aus PE, PP, PET, Rayon, Cellulose, PA und Mischungen dieser Fasern enthalten. Auch Bi- oder Multikomponentenfasern sind denkbar und vorteilhaft. Vorteilhaft sind insbesondere Kardenvliese, Spinnvliese, wasserstrahlvernadelte Vliese, SM-Vliese, SMS-Vliese, SMMS-Vliese oder auch Laminate aus einer oder mehreren dieser Vliesarten, wobei S für Spunbond- und M für Meltblown-Vliesschichten steht. Besonders bevorzugt sind Spinnvliese, da diese eine hohe Festigkeit in Längs- und Querrichtung aufweisen und somit den auf sie durch das Eingreifen von gegebenenfalls vorhandenen mechanischen Verschlusshilfen einwirkenden Scherkräften besonders gut standhalten können. Um zu verhindern, dass beim Lösen der mechanischen Verschlusshilfen Fasern aus dem Vliesverbund herausgerissen werden, ist vorteilhaft, die Vliesstoffkomponente mit einem Prägemuster zu versehen, vermittels dessen vorzugsweise alle Fasern der Vlieskomponente gebunden sind. Vorteilhaft ist solchenfalls insbesondere ein Thermoprägemuster, das insbesondere vorteilhaft durch Kalandern des Vliesstoffes unter Zuführung von thermischer Energie erzeugt wird.

Zur Herstellung eines erfindungsgemäßen gefalteten Inkontinenzartikels werden die hinteren Materialabschnitte vorzugsweise an mindestens zwei in Längsrichtung parallelen Falzlinien zickzackförmig auf sich selbst gefaltet und die gefalteten Materialabschnitte an vorzugsweise durch den Windelhauptteil verlaufenden Einschlagachsen nach innen auf die körperzugewandte Seite des Rückbereichs des Hauptteils eingeschlagen. Vorteilhaft könnte auch der Seitenrand des Hauptteils im Bereich der Anfügung der hinteren Materialabschnitte die Einschlagachsen bilden.

Entsprechend betrifft die Erfindung auch ein Verfahren zur Herstellung eines gefalteten Inkontinenzartikels nach mindestens einem der Ansprüche 1 bis 8, wobei die hinteren Materialabschnitte an in Längsrichtung parallelen Falzlinien vorzugsweise zickzackförmig auf sich selbst gefaltet werden und die gefalteten hinteren Materialabschnitte an den Einschlagachsen nach innen auf die körperzugewandte Seite des Rückbereichs des Hauptteils eingeschlagen werden, derart, dass die Innenseite des Randbereichs eines jeweiligen hinteren Materialabschnitts in Richtung auf die körperzugewandte Seite, also die Innenseite des Rückbereichs des Hauptteils orientiert ist. Vorzugsweise werden die Materialabschnitte zunächst auf sich selbst gefaltet und anschließend auf den Rückbereich des Hauptteils eingeschlagen. Weiterhin bevorzugt werden die Materialabschnitte zunächst auf sich selbst gefaltet, anschließend in gefalteter Konfiguration am Rückbereich des Hauptteil fixiert und dann auf die Innenseite des Hauptteils eingeschlagen.

In Weiterbildung dieses Erfindungsgedankens wird der Inkontinenzartikel mindestens einmal, vorzugsweise zweimal nach innen, vorzugsweise an in Querrichtung verlaufenden Faltlinien, auf sich selbst gefaltet, vorzugsweise derart, dass zunächst der Vorderbereich nach innen auf die Innenseite des Hauptteils und anschließend der Rückbereich auf den Vorderbereich gefaltet wird. Dadurch entsteht ein im Herstell- und Verpackungsprozess aufgrund seiner kompakten Größe gut handhabbares Produkt, dessen Sichtseiten in der zusammengefalteten Konfiguration durch die auch im Anwendungszustand die äußerste Lage bildende Materiallage gebildet wird, so dass die Innenseite des Artikels vor Gebrauch vor Verunreinigung geschützt ist.

Vorzugsweise erfolgt die Förderung der Inkontinenzartikel im Zuge der Herstellung in einer Fertigungsmaschine, insbesondere nach dem Verfahrensschritt des zickzackförmigen Faltens und Einschlagens der hinteren Materialabschnitte an den Einschlagachsen nach innen auf die körperzugewandte Seite des Rückbereichs des Hauptteils, parallel zur Längsrichtung und mit einer Bahngeschwindigkeit von mehr als 200 m/min, insbesondere mehr als 250 m/min, weiter insbesondere von mehr als 300 m/min, weiter insbesondere von mehr als 350 m/min. Vorzugsweise werden die Inkontinenzartikel hierbei bis einschließlich dieses vorgenannten Verfahrensschrittes noch endlos, also noch mit ihrem jeweiligen späteren Hüftöffnungsrändern aneinander verbunden gefördert. Vorzugsweise erst in einem nachgelagerten Verfahrensschritt werden die Inkontinenzartikel in Querrichtung zur Bildung der vereinzelten Inkontinenzartikel voneinander getrennt und nachfolgend wie zuvor beschrieben an in Querrichtung verlaufenden Faltlinien auf sich selbst gefaltet.

Weitere Merkmale, Einzelheiten und Vorteile der Erfindung ergeben sich aus den beigefügten Patentansprüchen, der zeichnerischen Darstellung und nachfolgenden Beschreibung einer bevorzugten Ausführungsform der Erfindung. In der Zeichnung zeigt:
Figur 1 eine Draufsicht auf einen vollständig entfalteten Inkontinenzartikel
Figur 2 eine Teildraufsicht auf einen erfindungsgemäß gefalteten Inkontinenzartikel mit auf sich selbst gefalteten hinteren Materialabschnitten
Figur 2a eine Schnittansicht des Inkontinenzartikels nach Figur 2 (A-A)
Figur 2b eine Schnittansicht des Inkontinenzartikels nach Figur 2 (B-B)
Figur 2c eine Schnittansicht des Inkontinenzartikels nach Figur 2 (A-A) in einer alternativen Ausführungsform
Figur 3a und 3b zeigen Schnittansichten eines an den Randbereich eines hinteren Materialabschnitts angefügten Verschlusselementes

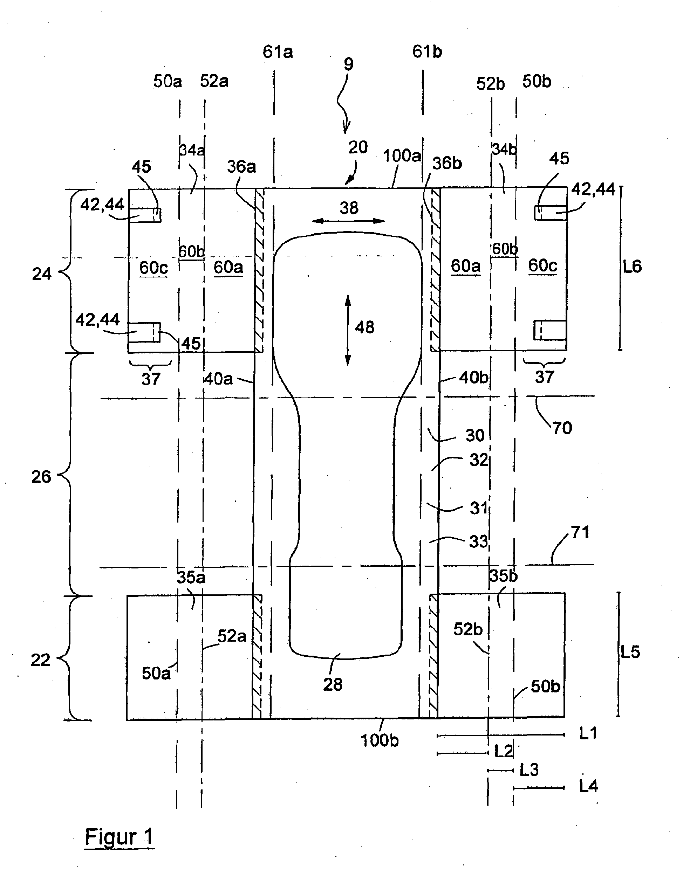

Ein erfindungsgemäßer Inkontinenzartikel 9 ist in den Figuren 1 bis 2b schematisch dargestellt. Er umfasst einen insgesamt mit dem Bezugszeichen 20 bezeichneten Hauptteil, der häufig auch als Chassis bezeichnet wird. Der Hauptteil 20 umfasst einen Vorderbereich 22, einen Rückbereich 24 und einen dazwischen liegenden Schrittbereich 26, der zwischen den Beinen eines Benutzers zu liegen kommt, wenn der Inkontinenzartikel 9 an einen Benutzer angelegt ist. Der Hauptteil 20 umfasst einen Saugkörper 28, der zur Aufnahme und dauerhaften Speicherung von Körperflüssigkeiten in geeigneter Weise dimensioniert ist. Der Saugkörper umfasst vorzugsweise Zellulosefasern und superabsorbierende Polymerpartikel (SAP) und ist von einer fiüssigkeitsundurchlässigen Schicht 30 unterfangen, welche auch die äußere Sichtseite des Inkontinenzartikels 9 bilden kann. Oberhalb des Saugkörpers 28 kann ein flüssigkeitsdurchlässiges Topsheet 32 vorgesehen sein.

Im Rückbereich 24 sind an einem ersten Seitenrandabschnitt 36a des Hauptteils 20 ein erster eine hintere Seitenklappe oder Seitenabschnitt bildender Materialabschnitt 34a und an einem zweiten Seitenrandabschnitt 36b des Hauptteils 20 ein zweiter eine hintere Seitenklappe oder Seitenabschnitt bildender Materialabschnitt 34b angefügt. Die Materialabschnitte 34a, 34b weisen eine rechteckförmige Kontur auf. Es wären auch Materialabschnitte 34a, 34b, die eine Konturierung am Beinausschnitt aufweisen, denkbar und vorteilhaft, wie in DE-102007024180-A1 offenbart. Die äußeren Querränder von Hauptteil 20 und den hinteren Materialabschnitten 34a, 34b bilden den hinteren Hüftöffnungsrand 100a.

Die im Anwendungszustand dem Körper des Trägers abgewandte Seite des Inkontinenzartikels 9 wird als Außenseite 31 bezeichnet, die im Anwendungszustand dem Körper des Trägers zugewandte Seite des Inkontinenzartikels 9 als Innenseite 33. Nach diesem Verständnis ist in der eben ausgefalteten Konfiguration des Inkontinenzartikels 9 jeder Komponente des Inkontinenzartikels 9 eine Innenseite und eine Außenseite zuzuordnen.

Figur 1 zeigt eine Sicht auf die Innenseite 33 des Inkontinenzartikels 9 im vollständig entfalteten Zustand. Die hinteren Materialabschnitte 34a, 34b tragen jeweils zwei Verschlusselemente 42 mit auf sich selbst gefalteten und zum bestimmungsgemäßen Gebrauch entfaltbaren Verschlusselementlaschen 44, welche freie Fingerlifte 45 aufweisen. Die Verschlusselementlaschen 44 wirken zum Schließen des Inkontinenzartikels in Gebrauch mit einer Außenseite 31 des Vorderbereichs 22 des Hauptteils 20 und/oder der Materialabschnitte im Vorderbereich 22 lösbar haftend zusammen. Hierzu können die Verschlusselementlaschen eine Haftkleberzone oder eine Zone mit mechanischen Verschlusshilfen wie Kletthaken aufweisen.

Im Vorderbereich 22 des Inkontinenzartikels sind ebenfalls auf sich selbst gefaltete Seitenabschnitte bildende Materialabschnitte 35a, 35b vorgesehen, die jedoch keine Verschlusselemente aufweisen. Die vorderen Materialabschnitte 35a, 35b weisen eine rechteckförmige Kontur auf. Es wären auch Materialabschnitte 35a, 35b, die eine Konturierung am Beinausschnitt aufweisen, denkbar und vorteilhaft, wie in DE-102007024180-A1 offenbart. Die äußeren Querränder von Hauptteil 20 und den vorderen Materialabschnitten 35a, 35b bilden den vorderen Hüftöffnungsrand 100b.

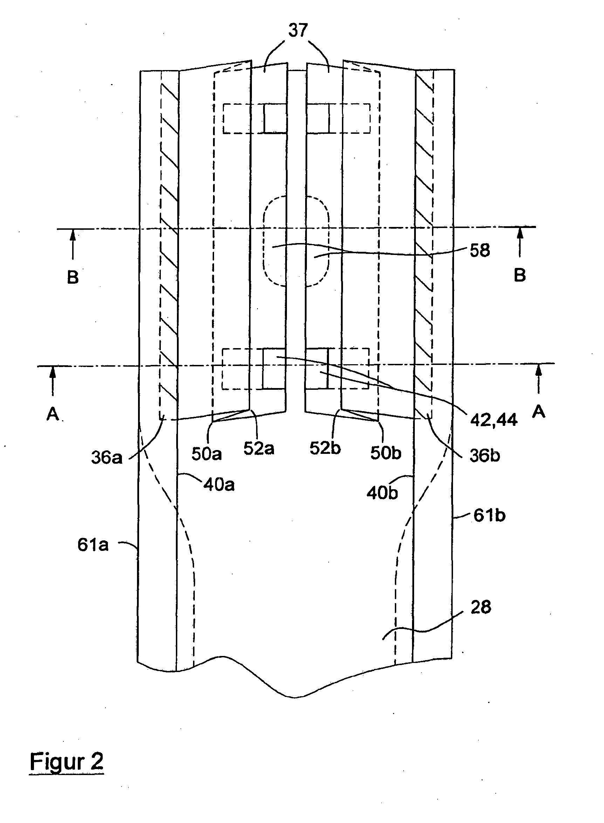

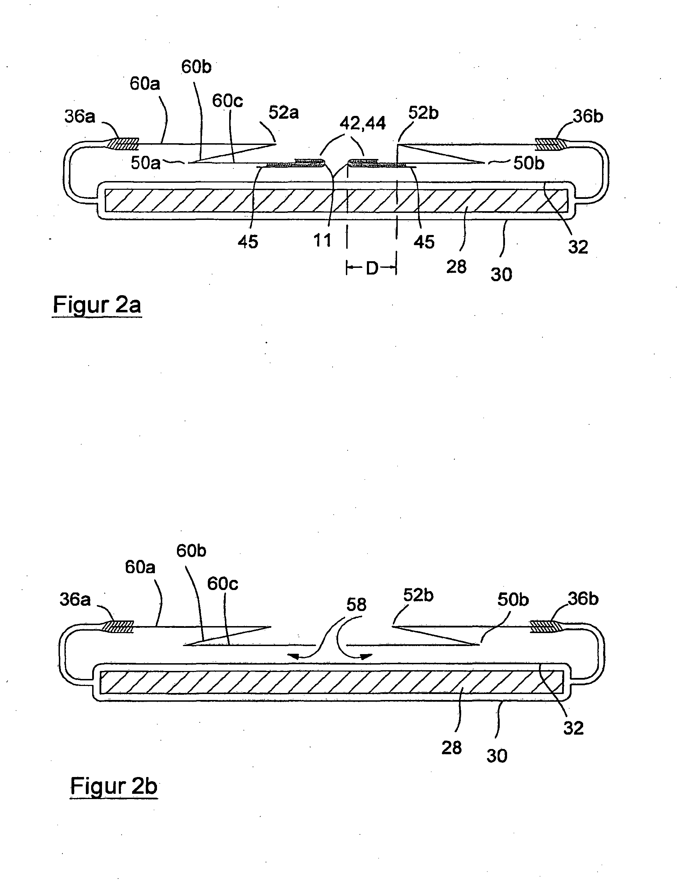

Der jeweilige hintere Materialabschnitt 34a, 34b im Rückbereich 24 des Hauptteils ist, wie aus der Figur 2a ersichtlich, entlang jeweils zweier in Längsrichtung 48 paralleler Falzlinien 50a, 52a bzw. 50b, 52b zickzackförmig auf sich selbst gefaltet, wobei Teilabschnitte 60a, 60b, 60c eines Materialabschnitts bereichsweise aufeinander zu liegen kommen.

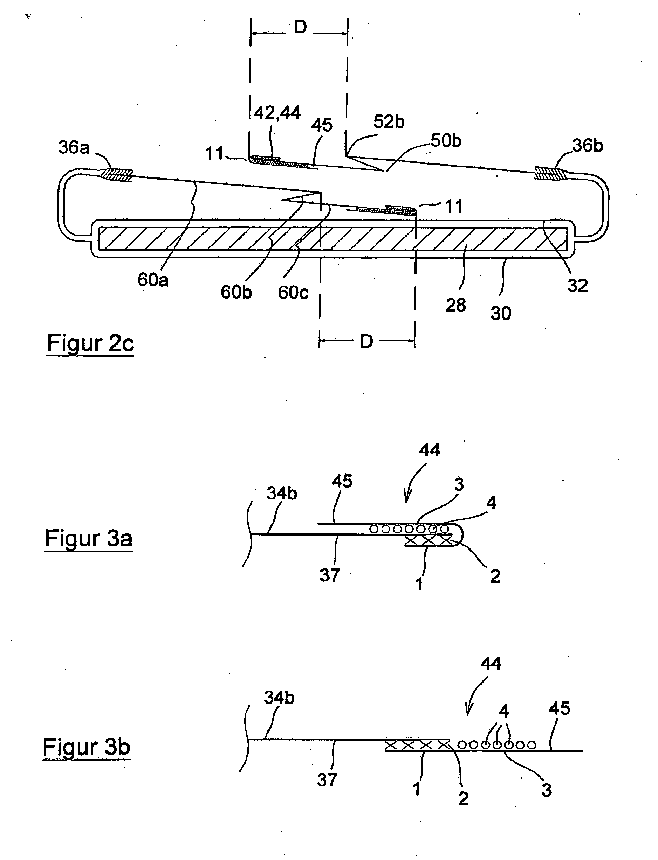

Im Zuge der Fertigung der Inkontinenzartikel 9 werden die jeweiligen hinteren Materialabschnitte 34a, 34b ausgehend von der Darstellung nach Figur 1 und nachdem sie zickzackförmig auf sich selbst gefaltet wurden, nach innen an jeweiligen zur Längsrichtung 48 parallelen Einschlagachsen 61a, 61b, die in der dargestellten Ausführungsform innerhalb des Hauptteils 20 verlaufen, zu einer eingeschlagenen Anordnung umgeschlagen in die in Figur 2 dargestellte Position. In dieser Anordnung werden die Inkontinenzartikel 9 im Zuge der Fertigung insbesondere noch endlos in der Längsrichtung mit einer hohen Bahngeschwindigkeit, insbesondere mit einer Bahngeschwindigkeit von mehr als 200 m/min, weiter insbesondere mit einer Bahngeschwindigkeit von mehr als 300 m/min, weiter insbesondere mit einer Bahngeschwindigkeit von mehr als 350 m/min gefördert. Solchenfalls wirkt auf die Oberseite der Bahn ein starker Luftzug ("Fahrtwind") ein. Dies birgt die Gefahr, dass eine starke Luftzug-Kraft auf die die Oberseite der Bahn bildenden Komponenten ausgeübt wird. Dadurch, dass ein äußerer Seitenrand 11 des zu unterst liegenden Teilabschnitts 60c eines hinteren Materialabschnitts 34a bzw. 34b gegenüber den darüber gefalteten Teilabschnitten 60a bzw. 60b in Querrichtung um das Maß D übersteht, bildet zumindest ein Teilbereich eines zu unterst liegenden Teilabschnittes 60c zumindest eines hinteren Materialabschnitts einen Teilbereich der Oberseite der Bahn. Die Erfinder haben erkannt, dass in dieser Konfiguration die Verschlusselementlaschen in besonderem Maße der Gefahr des zu Fertigungsfehlern führenden Einwirkens starker Luftzug-Kräfte ausgesetzt sind: Die Verschlusselementlasche 44 ist mit ihrem Herstellerende 1 üblicherweise unlösbar, das heißt sehr fest vorzugsweise mittels eines Permanentklebstoffes 2 oder auch mittels Thermobonding oder Ultraschallschweißen oder anderen Fügemethoden an einem Randbereich 37 der hinteren Materialabschnitte 34a, 34b fixiert (Figur 3a). An ihrem Benutzerende 3 hingegen ist die Verschlusselementlasche 44 üblicherweise zum Zwecke der späteren Verwendung zerstörungsfrei ablösbar, also mit einer geringen Kraft vom Anwender in den Gebrauchszustand entfaltbar fixiert, vorzugsweise mittels eines Haftklebstoffes 4 und/oder mittels mechanischer Verschlusselemente wie Kletthaken. Figur 3b zeigt die bestimmungsgemäß unmittelbar vor Gebrauch des Inkontinenzartikels entfaltete Verschlusselementlasche 44 nach dem zerstörungsfreien Ablösen von der Innenseite 33 des Randbereiches 37 eines hinteren Materialabschnitts 34a, 34b. Außerdem weist die Verschlusselementlasche 44 an einem äußersten Bereich ihres Benutzerendes 3 einen so genannten Fingerlift 45 auf, das heißt einen Endabschnitt, welcher zum Zwecke des einfachen Ergreifens durch den Anwender mit einer noch geringeren Kraft ablösbar ist, oder vorzugsweise, wie im dargestellten Fall, gänzlich unfixiert verbleibt.

In der erfindungsgemäßen Anordnung ist sichergestellt, dass das ablösbare Benutzerende 3 der Verschlusselementlasche 44 mitsamt seinem Fingerlift 45 in der gefalteten Anordnung in Richtung auf die körperzugewandte Seite, also auf die Innenseite 33 des Rückbereiches 24 orientiert also dieser zugewandt ist und somit nicht Teil der Oberseite der Bahn ist und somit nicht oder weniger stark den oben beschriebenen Luftzug-Kräften ausgesetzt ist. Stattdessen ist das permanent, also unlösbar fixierte Herstellerende 1 Teil der Oberseite der Bahn. Damit ist das Herstellerende 1 den Luftzug-Kräften in stärkerem Maße ausgesetzt, kann diesen aber besser widerstehen, da dessen Fixierung an dem Randbereich 37 des hinteren Materialabschnitts 34a, 34b bestimmungsgemäß unlösbar, also mit deutlich stärkerer Haftkraft erfolgt. Durch die erfindungsgemäße Anordnung ist außerdem sichergestellt, dass der Benutzer die hinteren Materialabschnitte 34a, 34b als solche erkennt, das heißt sie von den vorderen Seitenabschnitten unterscheiden kann und sie außerdem komfortabel ergreifen und entfalten kann, da ein zu unterst liegender gefalteter Teilabschnitt 60c des hinteren Materialabschnitts 34a, 34b den Randbereich 37 des Materialabschnitts umfasst und der Randbereich 37 die Verschlusselemente 44 aufweist und ein äußerer Seitenrand 11 des Teilabschnittes 60c gegenüber den darüber gefalteten Teilabschnitten 60a, 60b in Querrichtung 38 um das Maß D übersteht, sich also über die eigentliche Falte hinaus erstreckt.

Weiterhin erweist es sich als vorteilhaft, wenn an jedem der hinteren Materialabschnitte 34a, 34b an einem jeweiligen Randbereich 37 ein Anfassbereich 58 vorhanden ist (Figur 2b). Der Anfassbereich 58 ist der zum manuellen Ergreifen des gefalteten Materialabschnitts 34a, 34b geeignete Bereich, um diesen zu entfalten. Der jeweilige Anfassbereich 58 der Materialabschnitte 34a, 34b ist in der in Figur 2 dargestellten Konfiguration zu einer Längsmittelachse des Inkontinenzartikels 9 hin nach innen gewandt.

Weiterhin bevorzugt ist ein jeweiliger hinterer Materialabschnitt 34a, 34b derart gefaltet, dass ein äußerer Seitenrand 11 des zu unterst liegenden Teilabschnitts 60c eines hinteren Materialabschnitts 34a, 34b gegenüber den darüber gefalteten Teilabschnitten 60a, 60b in Querrichtung um das Maß D übersteht und damit für den Benutzer leichter zu greifen ist. D beträgt hierbei vorzugsweise mindestens 10 % und höchstens 90 % der Breite L4 des Teilabschnitts 60c, weiterhin bevorzugt mindestens 40 % und höchstens 60 %. Der Überstand um das Maß D beträgt vorzugsweise mindestens 6 mm und höchstens 54 mm, weiterhin bevorzugt mindestens 24 mm und höchstens 36 mm.

In einer bevorzugten Ausführungsform sind die aufeinander gefalteten Teilabschnitte 60a, 60b, 60c der Materialabschnitte 34a, 34b in der gefalteten Konfiguration insbesondere durch punktförmige durch Ultraschallschweißung erzeugte Fügestellen 62 vorzugsweise mit einem Durchmesser von 0,35 mm und einer Fläche von 0,0962 mm², die in Figur 2 dargestellt sind, vorzugsweise lösbar in dieser Konfiguration fixiert. Es hat sich gezeigt, dass diese lösbare Fixierung so gestaltet sein kann, dass sich durch einmaliges Ziehen an dem jeweiligen ersten Anfassbereich 58 der jeweilige Materialabschnitt 34a, 34b vollständig entfalten lässt, wobei vorzugsweise alle Fügestellen 62 gelöst oder aufgetrennt werden.

In der dargestellten bevorzugten Ausführungsform werden die in Längsrichtung aufeinander gefalteten hinteren Materialabschnitte 34a, 34b an den Einschlagachsen 61a, 61b derart auf die Innenseite des Rückbereichs 24 eingeschlagen, dass die Materialabschnitte 34a, 34b auf Stoß oder mit einem geringen Abstand voneinander, jedenfalls nicht einander überlappend zu liegen kommen (Figur 2a).

In einer alternativen Ausführungsform ist es jedoch denkbar und vorteilhaft, die in Längsrichtung aufeinander gefalteten hinteren Materialabschnitte 34a, 34b an den Einschlagachsen 61a, 61b derart weit auf die Innenseite des Rückbereichs 24 einzuschlagen, dass die Materialabschnitte 34a, 34b zumindest bereichsweise einander überlappend zu liegen kommen (Figur 2c).

Der Inkontinenzartikel 9 wird nach Fertigstellung der Längsfaltung der hinteren und vorderen Materialabschnitte und nach dem Einschlagen der gefalteten Materialabschnitte auf die Innenseite des Hauptteils vorzugsweise mindestens einmal, vorzugsweise zweimal nach innen, vorzugsweise an in Querrichtung 38 verlaufenden Faltlinien 70, 71 (Figur 1), auf sich selbst gefaltet, vorzugsweise derart, dass zunächst der Vorderbereich 22 nach innen auf die Innenseite 33 des Hauptteils 20 und anschließend der Rückbereich 24 auf den Vorderbereich 22 gefaltet wird. Dadurch entsteht ein im Herstell- und Verpackungsprozess aufgrund seiner kompakten Größe gut handhabbares Produkt, dessen Sichtseiten in der zusammengefalteten Konfiguration durch die auch im Anwendungszustand die äußerste Lage bildende Materiallage 30 gebildet wird, so dass die Innenseite 33 des Artikels 9 vor Gebrauch vor Verunreinigung geschützt ist.

Anhand der Figur 1 sind die Abmessungen der Teilabschnitte 60a, 60b, 60c des jeweiligen Materialabschnitts in der dargestellten Ausführungsform veranschaulicht. Die Gesamtbreite L1 in Querrichtung eines entfalteten vorderen und hinteren Materialabschnitts beträgt in Querrichtung 38 165 mm. Die Breite L2 des an den Hauptteil 20 angrenzenden Teilabschnitts 60a beträgt ca. 70 mm. Die Breite L3 des mittleren Teilabschnitts 60b beträgt ca. 35 mm, und die Breite L4 des äußeren, den Randbereich 37 umfassenden Teilabschnitts 60c beträgt ca. 60 mm. Die Länge der Materialabschnitte im Vorderbereich L5 beträgt 165 mm, die Länge der Materialabschnitte im Rückbereich L6 beträgt 230 mm.

## Patentansprüche

1. Absorbierender Inkontinenzartikel (9) mit einem Hauptteil (20), bestehend aus einem Vorderbereich (22), einem Rückbereich (24) und einem in Längsrichtung (48) dazwischen liegenden zwischen den Beinen eines Benutzers zu liegen kommenden Schrittbereich (26), wobei der Hauptteil (20) einen Saugkörper (28) umfasst, und mit an den Rückbereich (24) angefügten hinteren Materialabschnitten (34a, 34b) und mit an den Vorderbereich (22) angefügten vorderen Materialabschnitten (35a, 35b), wobei sich die hinteren und vorderen Materialabschnitte (34a, 34b, 35a, 35b) in Querrichtung (38) über seitliche Längsränder (40a, 40b) des Hauptteils (20) hinaus erstrecken, wobei hintere und vordere Materialabschnitte (34a, 34b, 35a, 35b) den Vorderbereich (22) und den Rückbereich (24) im Anwendungszustand des Artikels (9) miteinander verbinden, wobei die hinteren und vorderen Materialabschnitte (34a, 34b, 35a, 35b) eine im Anwendungszustand auf der körperzugewandten Seite liegende Innenseite (33) und eine im Anwendungszustand auf der körperabgewandten Seite liegende Außenseite (31) aufweisen und wobei hintere Materialabschnitte (34a, 34b) an einem äußeren Randbereich (37) der Materialabschnitte (34a, 34b) Verschlusselemente (42) aufweisen, wobei die Verschlusselemente (42) eine Verschlusselementlasche (44) mit einem freien Fingerlift (45) umfassen, welche vor Gebrauch auf die Innenseite des Materialabschnitts zurückgefaltet ist, wobei diese Materialabschnitte (34a, 34b) vor Ingebrauchnahme des Artikels (9) zickzackförmig auf sich selbst und nach innen auf die körperzugewandte Seite des Rückbereichs des Hauptteils (20) zu einer gefalteten Anordnung gefaltet sind an zu der in Längsrichtung (48) parallel verlaufenden Falzlinien (50a, 50b, 52a, 52b) derart, dass die Falzlinien die hinteren Materialabschnitte (34a, 34b) in Teilabschnitte unterteilen und in der gefalteten Anordnung ein zu unterst liegender gefalteter Teilabschnitt (60c) der Materialabschnitte den Randbereich (37) umfasst und die Innenseite des Randbereichs (37) in Richtung auf die Innenseite (33) des Rückbereichs (24) des Hauptteils (20) orientiert ist, wobei ein jeweiliger hinterer Materialabschnitt (34a, 34b) derart gefaltet ist, dass ein äußerer Seitenrand (11) des zu unterst liegenden Teilabschnitts (60c) eines hinteren Materialabschnitts (34a, 34b) gegenüber den darüber gefalteten Teilabschnitten (60a, 60b) in Querrichtung um das Maß D übersteht.

2. Absorbierender Inkontinenzartikel (9) gemäß Anspruch 1, **dadurch gekennzeichnet, dass** das Maß D vorzugsweise mindestens 10 % und höchstens 90 % der Breite (L4) des zu unterst liegenden Teilabschnitts (60c), weiterhin bevorzugt mindestens 40 % und höchstens 60 % beträgt oder der Überstand um das Maß D vorzugsweise mindestens 6 mm und höchstens 54 mm, weiterhin bevorzugt mindestens 24 mm und höchstens 36 mm beträgt.

3. Absorbierender Inkontinenzartikel (9) gemäß einem der vorangehenden Ansprüche, **dadurch gekennzeichnet, dass** die Falzlinien die hinteren Materialabschnitte (34a, 34b) in drei Teilabschnitte unterteilen, wobei die Breite (L3) des mittleren Teilabschnitts kleiner ist als die Breiten (L2, L4) der zum mittleren Teilabschnitt benachbarten Teilabschnitte.

4. Absorbierender Inkontinenzartikel (9) gemäß einem der vorangehenden Ansprüche, **dadurch gekennzeichnet, dass** die Falzlinien die hinteren Materialabschnitte (34a, 34b) in drei Teilabschnitte unterteilen mit einem Verhältnis der Breite (L2, L3, L4) der Teilabschnitte (60a, 60b, 60c) von 2:1:2.

5. Absorbierender Inkontinenzartikel (9) gemäß einem der vorangehenden Ansprüche, **dadurch gekennzeichnet, dass** an jedem der hinteren Materialabschnitte (34a, 34b) an einem Randbereich (37) ein Anfassbereich (58) vorhanden ist.

6. Absorbierender Inkontinenzartikel (9) gemäß einem der vorangehenden Ansprüche 1-5, **dadurch gekennzeichnet, dass** in Längsrichtung (48) aufeinander gefaltete hintere Materialabschnitte (34a, 34b) an den Einschlagachsen (61 a, 61 b) derart auf die Innenseite des Rückbereichs (24) eingeschlagen sind, dass die Materialabschnitte (34a, 34b) zumindest bereichsweise einander überlappend oder auf Stoß oder mit einem geringen Abstand voneinander zu liegen kommen.

7. Absorbierender Inkontinenzartikel (9) gemäß einem der vorangehenden Ansprüche, **dadurch gekennzeichnet, dass** die vorderen Materialabschnitte (35a, 35b) vor Ingebrauchnahme des zusammengefalteten Artikels (9) auf sich selbst gefaltet sind an zu der Längsrichtung (48) parallel verlaufenden Falzlinien (50a, 50b, 52a, 52b).

8. Absorbierender Inkontinenzartikel (9) gemäß einem der vorangehenden Ansprüche, **dadurch gekennzeichnet, dass** die vorderen Materialabschnitte (35a, 35b) vor Ingebrauchnahme des zusammengefalteten Artikels (9) zickzackförmig auf sich selbst gefaltet sind an zu der Längsrichtung (48) parallel verlaufenden Falzlinien (50a, 50b, 52a, 52b) derart, dass die Falzlinien die vorderen Materialabschnitte (35a, 35b) in drei Teilabschnitte unterteilen mit einem Verhältnis der Breite (L2, L3, L4) der Teilabschnitte (60a, 60b, 60c) von 2:1:2.

9. Verfahren zur Herstellung eines gefalteten Inkontinenzartikels (9) gemäß einem der vorangehenden Ansprüche, **dadurch gekennzeichnet, dass** die hinteren Materialabschnitte (34a, 34b) an in Längsrichtung (48) parallelen Falzlinien (50a, 52a bzw. 50b, 52b) zickzackförmig auf sich selbst gefaltet werden und dass die gefalteten hinteren Materialabschnitte an den Einschlagachsen (61 a, 61 b) nach innen auf die körperzugewandte Seite des Rückbereichs des Hauptteils (20) eingeschlagen werden, derart, dass die Innenseite des Randbereichs eines jeweiligen hinteren Materialabschnitts in Richtung auf die Innenseite (33) des Rückbereichs (24) des Hauptteils (20) orientiert ist.

10. Verfahren zur Herstellung gefalteter Inkontinenzartikels (9) gemäß Anspruch 9, **dadurch gekennzeichnet, dass** die Förderung der Inkontinenzartikel (9) in einer Fertigungsmaschine parallel zur Längsrichtung (48) mit einer Bahngeschwindigkeit von mehr als 200 m/min, insbesondere mehr als 250 m/min, weiter insbesondere von mehr als 300 m/min, weiter insbesondere von mehr als 350 m/min erfolgt.

11. Verfahren zur Herstellung gefalteter Inkontinenzartikel (9) gemäß Anspruch 9 oder 10, **dadurch gekennzeichnet, dass** die Inkontinenzartikel (9) mindestens einmal, vorzugsweise zweimal nach innen, vorzugsweise an in Querrichtung (38) verlaufenden Faltlinien (70, 71), auf sich selbst gefaltet werden, vorzugsweise derart, dass zunächst der Vorderbereich (22) auf die Innenseite des Hauptteils und anschließend der Rückbereich (24) auf die Außenseite des Vorderbereichs gefaltet wird.

## Claims

1. Absorbent incontinence article (9) having a main part (20), made up of a front region (22), of a rear region (24) and of a crotch region (26) which is located therebetween, as seen in the longitudinal direction (48), and ends up between a user's legs, wherein the main part (20) comprises an absorbent body (28), and having rear material portions (34a, 34b), which are attached to the rear region (24), and having front material portions (35a, 35b), which are attached to the front region (22), wherein the rear and front material portions (34a, 34b, 35a, 35b) extend in the transverse direction (38) beyond lateral longitudinal peripheries (40a, 40b) of the main part (20), wherein rear and front material portions (34a, 34b, 35a, 35b) connect the front region (22) and the rear region (24) to one another in the use state of the article (9), wherein the rear and front material portions (34a, 34b, 35a, 35b) have an inside (33), which in the use state faces the body, and an outside (31), which in the use state faces away from the body, and wherein rear material portions (34a, 34b) have closure elements (42) on an outer peripheral region (37) of the material portions (34a, 34b), wherein the closure elements (42) comprise a closure-element tab (44) which has a free finger lift (45) and, prior to use, is folded back onto the inside of the material portion, wherein these material portions (34a, 34b), prior to initial use of the article (9), are folded onto themselves in zigzag form and in the inward direction, onto that side of the rear region of the main part (20) which faces the body, to give a folded arrangement along folding lines (50a, 50b, 52a, 52b) running parallel to the longitudinal direction (48), such that the folding lines subdivide the rear material portions (34a, 34b) into sub-portions and, in the folded arrangement, a lowermost folded sub-portion (60c) of the material portions comprises the peripheral region (37) and the inside of the peripheral region (37) is oriented in the direction of the inside (33) of the rear region (24) of the main part (20), wherein a resepective rear material portion (34a, 34b) is folded such that an outer side periphery (11) of the lowermost sub-portion (60c) of a rear material portion (34a, 34b) projects in the transverse direction by the extent D in relation to the sub-portions (60a, 60b) folded above the same.

2. Absorbent incontinence article (9) according to Claim 1, **characterized in that** the extent D is prefereably at least 10%, and at most 90%, of the width (L4) of the lowermost sub-portion (60c), further preferably at least 40%, and at most 60%, or the projection by the extent D is preferably at least 6 mm and at most 54 mm, further preferably at least 24 mm and at most 36 mm.

3. Absorbent incontinence article (9) according to either of the preceding claims, **characterized in that** the folding lines subdivide the rear material portions (34a, 34b) into three sub-portions, wherein the width (L3) of the middle sub-portion is smaller than the widths (L2, L4) of the sub-portions adjacent to the middle sub-portion.

4. Absorbent incontinence article (9) according to one of the preceding claims, **characterized in that** the folding lines subdivide the rear material portions (34a, 34b) into three sub-portions with a ratio of the widths (L2, L3, L4) of the sub-portions (60a, 60b, 60c) of 2:1:2.

5. Absorbent incontinence article (9) according to one of the preceding claims, **characterized in that** a gripping region (58) is present on a peripheral region (37) on each of the rear material portions (34a, 34b).

6. Absorbent incontinence article (9) according to one of the preceding Claims 1-5, **characterized in that** rear material portions (34a, 34b) folded one upon the other in the longitudinal direction (48) are folded in onto the inside of the rear region (24), along the folding-in axes (61a, 61b), such that the material portions (34a, 34b) end up overlapping one another at least in certain regions or in abutment, or at a small spacing apart from one another.

7. Absorbent incontinence article (9) according to one of the preceding claims, **characterized in that** the front material portions (35a, 35b), prior to initial use of the folded-together article (9), are folded onto themselves along folding lines (50a, 50b, 52a, 52b) running parallel to the longitudinal direction (48).

8. Absorbent incontinence article (9) according to one of the preceding claims, **characterized in that** the front material portions (35a, 35b), prior to initial use of the folded-together article (9), are folded onto themselves in zigzag form along folding lines (50a, 50b, 52a, 52b) running parallel to the longitudinal direction (48), such that the folding lines subdivide the front material portions (35a, 35b) into three sub-portions with a ratio of the widths (L2, L3, L4) of the sub-portions (60a, 60b, 60c) of 2:1:2.

9. Method of producing a folded incontinence article (9) according to one of the preceding claims, **characterized in that** the rear material portions (34a, 34b) are folded onto themselves in zigzag form along folding lines (50a, 52a and 50b, 52b, respectively) parallel in the longitudinal direction (48), and **in that** the folded rear material portions are folded in in the inward direction onto that side of the rear region of the main part (20) which faces the body, along the folding-in axes (61a, 61b), such that the inside of the peripheral region of a respective rear material portion is oriented in the direction of the inside (33) of the rear region (24) of the main part (20).

10. Method of producing folded incontinence articles (9) according to Claim 9, **characterized in that** the incontinence articles (9) are conveyed in a production machine, parallel to the longitudinal direction (48), at a web speed of more than 200 m/min, in particular more than 250 m/min, further in particular of more than 300 m/min, and furthermore in particular of more than 350 m/min.

11. Method of producing folded incontinence articles (9) according to Claim 9 or 10, **characterized in that** the incontinence articles (9) are folded onto themselves at least once, preferably twice, in the inward direction, preferably along folding lines (70, 71) running in the transverse direction (38), preferably such that in a first instance the front region (22) is folded onto the inside of the main part and then the rear region (24) is folded onto the outside of the front region.

## Revendications

1. Article absorbant pour incontinence (9) comportant une partie principale (20), constituée d'une région avant (22), d'une région arrière (24) et d'une région de fourche (26) située entre elles dans la direction longitudinale (48), venant se positionner entre les jambes d'un utilisateur, la partie principale (20) comprenant un corps absorbant (28), et comportant des sections de matériau arrière (34a, 34b) raccordées à la région arrière (24) et comportant des sections de matériau avant (35a, 35b) raccordées à la région avant (22), les sections de matériau arrière et avant (34a, 34b, 35a, 35b) s'étendant dans la direction transversale (38) au-delà de bords longitudinaux latéraux (40a, 40b) de la partie principale (20), les sections de matériau arrière et avant (34a, 34b, 35a, 35b) reliant l'une à l'autre la région avant (22) et la région arrière (24) dans l'état d'utilisation de l'article (9), les sections de matériau arrière et avant (34a, 34b, 35a, 35b) présentant un côté intérieur (33) situé du côté tourné vers le corps dans l'état d'utilisation et un côté extérieur (31) situé du côté opposé au corps dans l'état d'utilisation et les sections de matériau arrière (34a, 34b) présentant des éléments de fermeture (42) au niveau d'une région de bord extérieure (37) des sections de matériau (34a, 34b), les éléments de fermeture (42) comprenant une patte d'élément de fermeture (44) avec une languette libre fingerlift (45), qui est repliée avant l'utilisation sur le côté intérieur de la section de matériau, ces sections de matériau (34a, 34b) étant repliées sur elles-mêmes en zigzag avant l'utilisation de l'article (9) et vers l'intérieur sur le côté tourné vers le corps de la région arrière de la partie principale (20) pour former un agencement plié au niveau de lignes de pliage (50a, 50b, 52a, 52b) s'étendant parallèlement dans la direction longitudinale (48) de telle sorte que les lignes de pliage divisent les sections de matériau arrière (34a, 34b) en sections partielles, et dans l'agencement plié, une section partielle pliée (60c) située en position la plus inférieure des sections de matériau comprenant la région de bord (37) et le côté intérieur de la région de bord (37) étant orienté dans la direction du côté intérieur (33) de la région arrière (24) de la partie principale (20), une section de matériau arrière respective (34a, 34b) étant pliée de telle sorte qu'un bord latéral extérieur (11) de la section partielle (60c) située en position la plus inférieure d'une section de matériau arrière (34a, 34b) dépasse d'une dimension D dans la direction transversale par rapport aux sections partielles pliées par-dessus (60a, 60b).

2. Article absorbant pour incontinence (9) selon la revendication 1, **caractérisé en ce que** la dimension D vaut de préférence au moins 10 % et au maximum 90 % de la largeur (L4) de la section partielle située en position la plus inférieure (60c), et de préférence au moins 40 % et au maximum 60 %, ou le dépassement de la dimension D vaut de préférence au moins 6 mm et au maximum 54 mm, et de préférence au moins 24 mm et au maximum 36 mm.

3. Article absorbant pour incontinence (9) selon l'une quelconque des revendications précédentes, **caractérisé en ce que** les lignes de pliage divisent les sections de matériau arrière (34a, 34b) en trois sections partielles, la largeur (L3) de la section partielle centrale étant inférieure aux largeurs (L2, L4) des sections partielles adjacentes à la section partielle centrale.

4. Article absorbant pour incontinence (9) selon l'une quelconque des revendications précédentes, **caractérisé en ce que** les lignes de pliage divisent les sections de matériau arrière (34a, 34b) en trois sections partielles, avec un rapport de la largeur (L2, L3, L4) des sections partielles (60a, 60b, 60c) de 2:1:2.

5. Article absorbant pour incontinence (9) selon l'une quelconque des revendications précédentes, **caractérisé en ce qu'**au niveau de chacune des sections de matériau arrière (34a, 34b), une région de saisie (58) est prévue au niveau d'une région de bord (37).

6. Article absorbant pour incontinence (9) selon l'une quelconque des revendications précédentes 1 à 5, **caractérisé en ce que** des sections de matériau arrière (34a, 34b) pliées les unes sur les autres dans la direction longitudinale (48) sont rabattues au niveau des axes de rabattement (61a, 61b) de telle sorte sur le côté intérieur de la région arrière (24) que les sections de matériau (34a, 34b) viennent s'appliquer au moins en partie en se chevauchant mutuellement ou en aboutement ou avec une légère distance entre elles.

7. Article absorbant pour incontinence (9) selon l'une quelconque des revendications précédentes, **caractérisé en ce que** les sections de matériau avant (35a, 35b), avant l'utilisation de l'article replié (9), sont repliées sur elles-mêmes au niveau de lignes de pliage (50a, 50b, 52a, 52b) s'étendant parallèlement à la direction longitudinale (48).

8. Article absorbant pour incontinence (9) selon l'une quelconque des revendications précédentes, **caractérisé en ce que** les sections de matériau avant (35a, 35b), avant l'utilisation de l'article replié (9), sont repliées sur elles-mêmes en zigzag et au niveau de lignes de pliage (50a, 50b, 52a, 52b) s'étendant parallèlement à la direction longitudinale (48), de telle sorte que les lignes de pliage divisent les sections de matériau avant (35a, 35b) en trois sections partielles avec un rapport de la largeur (L2, L3, L4) des sections partielles (60a, 60b, 60c) de 2:1:2.

9. Procédé de fabrication d'un article pour incontinence plié (9) selon l'une quelconque des revendications précédentes, **caractérisé en ce que** les sections de matériau arrière (34a, 34b) sont pliées sur elles-mêmes en zigzag au niveau de lignes de pliage (50a, 52a, respectivement 50b, 52b) parallèles dans la direction longitudinale (48) et **en ce que** les sections de matériau arrière pliées sont rabattues au niveau des axes de rabattement (61a, 61b) vers l'intérieur sur le côté tourné vers le corps de la région arrière de la partie principale (20) de telle sorte que le côté intérieur de la région de bord d'une section de matériau arrière respective soit orienté dans la direction du côté intérieur (33) de la région arrière (24) de la partie principale (20).

10. Procédé de fabrication d'articles pour incontinence pliés (9) selon la revendication 9, **caractérisé en ce que** le transport des articles pour incontinence (9) s'effectue dans une machine de fabrication parallèlement à la direction longitudinale (48) avec une vitesse de bande supérieure à 200 m/min, en particulier supérieure à 250 m/min, plus particulièrement supérieure à 300 m/min, et tout particulièrement supérieure à 350 m/min.

11. Procédé de fabrication d'articles pour incontinence pliés (9) selon la revendication 9 ou 10, **caractérisé en ce que** les articles pour incontinence (9) sont pliés sur eux-mêmes au moins une fois, de préférence deux fois, vers l'intérieur, de préférence au niveau de lignes de pliage (70, 71) s'étendant dans la direction transversale (38), de préférence de telle sorte que la région avant (22) soit d'abord pliée sur le côté intérieur de la partie principale et qu'ensuite la région arrière (24) soit pliée sur le côté extérieur de la région avant.
